# EUROPEAN PATENT SPECIFICATION

(11) **EP 3 198 027 B1**
(45) Date of publication and mention of the grant of the patent: **30.01.2019**
(21) Application number: 15766861.7
(22) Date of filing: 23.09.2015
(51) Int. Cl.: C12Q 1/6837

(54) **FAST AND HIGHLY SPECIFIC DNA-BASED MULTIPLEX DETECTION**
SCHNELLE UND HOCHSPEZIFISCHE DNA-BASIERTE MULTIPLEXDETEKTION
DÉTECTION MULTIPLEX À BASE D'ADN HAUTEMENT SPÉCIFIQUE ET RAPIDE

(30) Priority: 23.09.2014 EP 14185938
(43) Date of publication of application: 02.08.2017
(73) Proprietor: AIT Austrian Institute of Technology GmbH, 1220 Wien (AT)
(72) Inventor: BARISIC, Ivan, 1150 Wien (AT)
(74) Representative: Gassner, Birgitta
(86) International application number: PCT/EP2015/071827
(87) International publication number: WO 2016/046253

(56) References cited:
- EP-A1- 2 186 909
- WO-A1-00/04193
- WO-A1-98/31836

## Description

### Field of the Invention

Disclosed herein are methods and reagents for multiplex detection of target DNA. Specifically the invention provides a solid substrate comprising a plurality of DNA probes, wherein each probe contains at least two linear nucleotide chains (LNCs) linked together by a complementary nucleotide region and wherein one LNC having a nucleotide region complementary to another LNC is linked to said substrate and one LNC comprises a nucleotide region complementary to a further LNC and a nucleotide region complementary to a target sequence and optionally at least one further LNC comprises two oligonucleotide regions complementary to LNCs.

### Background Art

Highly specific and fast multiplex detection methods are essential to conduct reasonable DNA based diagnostics and are especially important to characterise infectious diseases. More than 1000 genetic targets such as antibiotic resistance genes, virulence factors and phylogenetic markers have to be identified as fast as possible to facilitate the correct treatment of a patient.

Solid support based nucleic acid detection methods allow a high degree of multiplexing due to the possibility to spatially separate DNA oligonucleotides, and thus, reduce unintended hybridisation events. Microarrays can detect several 100,000 targets simultaneously. However, the frequent unspecific hybridisation of dissolved DNA to immobilised oligonucleotides represents a significant limitation of the technique. A wide range of support materials, spacers, detection principles and labels were proposed to improve microarray detection but had limited success. Thus, various enzymatic reactions were combined with the microarray technology to enhance the specificity and sensitivity. The proof-reading activity of the used enzymes was meant to improve the specificity. Furthermore, the enzymatic reactions should multiply the signal intensity.

The on-chip PCR using primers immobilised to a surface was the first enzymatic driven method taking advantage of the relatively new microarray technology. The genomic DNA hybridises to the immobilised primers and is amplified analogous to a liquid phase PCR in a process also referred to as bridge amplification. Today, the method is used to amplify the total DNA in the next-generation sequencers. Since a few decades efforts are made to combine the microarray technology with enzymatic reactions to improve the sensitivity and specificity of the microarrays. The immobilisation of the PCR reaction to a surface was among the first approaches to take advantage of the multiplexing capability of microarrays. The solid-phase PCR is analogous to the conventional PCR but uses immobilised primer pairs that are elongated by a polymerase. Although huge effort has been put in the optimisation of the reaction parameters of the on-chip PCR, a highly specific multiplex amplification could never be achieved so far. Various surface chemistries, oligonucleotide spacers, primer lengths, buffer compositions, etc. were tested. The majority of the studies presented only proof-of-concepts or integrated statistical classifiers based on pattern recognition of the fluorescence signals but failed to demonstrate the specific amplification in a multiplex reaction. The sequencing technology of Illumia's Solexa is based on the same reaction mechanism also referred to as bridge amplification. However, the unspecific hybridisation and amplification of target DNA to the immobilised primers represents no drawback for this technology because the whole DNA is amplified and sequenced subsequently.

Thus, despite huge efforts optimising a vast range of parameters, the specificity of the on-chip PCR remained unsatisfactory. Therefore, other enzymes such as ligases with superior proof-reading capabilities were combined with microarrays.

In contrast to PCR, ligase dependent liquid phase assays detect DNA targets very specifically in multiplex reactions. Several works were presented where the identification of up to thousand different genetic targets was demonstrated using a single test. As a consequence, ligation based methods were adapted, and dissolved oligonucleotides were replaced by immobilised ones. The most simple detection principle used fluorescently labelled detection oligonucleotides that were ligated in the presence of a DNA template to the immobilised probe. Due to the lack of an amplification step, the sensitivity of such approaches was low. Thus, ligation based reactions with a subsequent rolling circle amplification (RCA) were introduced. However, to our best knowledge, none of the solid support based methods could be used for the specific detection of various targets in a multiplex reaction.

EP 2 186 909 discloses a method and apparatus for real-time, simultaneous, quantitative measurement of one or more single nucleotide polymorphisms in one or more target nucleic acids is provided. This method involves combining a ligase chain reaction (LCR), a ligase detection reaction (LDR), and/or a polymerase chain reaction (PCR) technique with an evanescent wave technique.

WO 00/04193 describes a method for detecting single nucleic acid molecules using rolling circle amplification (RCA) of single-stranded circular templates. In the presence of a nucleic acid molecule having the target sequence, a probe and a combination probe/primer oligonucleotide can hybridize to adjacent sites on the target sequence allowing the probes to be ligated together. By attaching the first probe to a substrate such as a bead or glass slide, unligated probe/primer can be removed after ligation. The method is useful for determining which target sequences are present in a nucleic acid sample, or for determining which samples contain a target sequence.

WO 98/31836 discloses a method for detecting a target nucleic acid species including the steps of providing an array of probes affixed to a substrate and a plurality of labeled probes.

The need for sensitive multiplex DNA detection methods is high, especially in clinical microbiology. Thousands of different antibiotic resistance mechanisms, virulence factors and pathogenic species have to be identified. Cultivation based methods are cost efficient but need up to a few weeks to obtain the first results after sampling. Additionally, some pathogens such as various intracellular bacteria cannot be cultivated easily at all. In contrast, DNA based diagnostics provide the clinicians with potentially lifesaving information within a few hours.

There is hence a need for improved means and methods for an efficient multiplex detection method allowing for a fast and reliable detection of target molecules, specifically of pathogens.

### Summary of invention

The invention is defined by the appended claims.

The present invention addresses this need and provides means and methods, which allow an efficient capture of target nucleic acid molecules on microarrays.

The inventors developed a very fast, simple, highly specific and sensitive multiplex detection method adaptable for a wide range of applications.

The DNA hybridisation behaviour of the immobilised oligonucleotide is considered as key parameter to achieve a good specificity. Thus, the oligonucleotide involved in the detection of the genetic target was not covalently attached to the surface but over a DNA chain structure formed via molecular attractive interactions. The goal was to emulate the hybridisation behaviour of the DNA oligonucleotides involved in the liquid phase multiplex ligation assays.

These molecular attractive interactions can be caused by hydrogen bridge bounds, other electrostatic interactions or van der Waals forces.

Disclosed herein is a novel ligation-based DNA probe concept that was combined with the microarray technology and used it for the detection of pathogens, specifically of bacterial pathogens. The novel probes identified all tested species correctly within one hour based on their 16S rRNA gene in a 20-multiplex reaction. Genomic DNA was used directly as template in the ligation reaction identifying as little as 10⁷ cells without any pre-amplification. The extremely high specificity was further demonstrated by characterising a single nucleotide polymorphism (SNP) leading to no false positive fluorescence signals of the untargeted SNP variants. In comparison to conventional microarray probes, the sensitivity of the novel LNC probes was higher by a factor of 10 or more.

Further disclosed is a solid support based detection platform targeting selected intracellular pathogens and clinically most prevalent bacterial pathogens, specifically *Escherichia coli, Klebsiella pneumoniae* and *Staphylococcus aureus.*

The objective is, in particular, accomplished by a solid substrate comprising a plurality of DNA probes, wherein each probe contains a number of linear nucleotide chains (LNCs) linked together by complementary nucleotide regions, wherein
1. a first LNC is immobilized to said substrate via the 5' end and is linked to a second LNC, and
2. the second LNC is linked to one or more further LNC(s) via complementary nucleotide regions, and wherein
3. the terminal LNC comprises a nucleotide sequence of interest.

In a preferred embodiment of the invention, the LNC is immobilized on the solid substrate covalently or via hydrogen bonds, specifically the immobilized LNC contains a thiol-modified 5'-end.

In a further embodiment, the LNCs contain at least one spacer region between the complementary regions, preferably said spacer regions are of three to ten nucleotides length, preferably three to six nucleotides. Said spacer regions can comprise any nucleotide sequence, specifically they are of a sequence of identical nucleotides, i.e. adenine, thymine, cytosine or guanine, preferably said nucleotides are thymines. In a further embodiment, the LNCs contain a spacer region of 1, 2, 3, 4, 5, 6, 7, 8, 9, 10, 11, 12, 13, 14, 15 nucleotides length.

In a further embodiment, the LNC probes contain at least 50%, preferably at least 60%, preferably at least 70%, more preferably at least 80% guanines and cytosines.

In yet a further embodiment of the invention, one of the LNCs containing a fluorescence conjugate for assessing the spotting quality and subsequent target quantification.

In a further embodiment, the target complementary sequence is a complementary antisense nucleotide sequence to a target sequence of interest, specifically of pathogen sequences, antibiotic resistance sequences, virulence factor sequences.

According to a specific embodiment of the invention, the LNCs are connected via hydrogen bounds to form a double helical strand.

Specifically, said probe comprises three to ten LNCs, preferably three to five LNCs, more preferred three LNCs.

In a preferred embodiment, the complementary region comprises 10 to 50 nucleotides, specifically 15 to 40 nucleotides, specifically 20 to 30 nucleotides, more specifically about 25 nucleotides.

The substrate may be selected from the group of glass, plastic, ceramic substrates, nanoparticles, magnetic or non-magnetic beads, gels.

The present invention further provides a biochip or comprising a substrate with a plurality of probes.

Additionally, disclosed is also a method for producing a biochip, comprising the steps of
providing a substrate;
preparing the probes; and
spotting the probes on the substrate.

A kit comprising a biochip according to the invention and a detection oligonucleotide is provided, specifically said detection oligonucleotide is labelled.

The invention also embodies the use of the biochip for the detection and/or identification and characterization of pathogens, viruses, for genetic analysis of prokaryotic and eukaryotic cells.

In yet a further embodiment of the invention, a method for detecting a DNA of interest is provided, comprising the steps of providing a biochip; adding a target DNA and a labelled detection oligonucleotide;
hybridizing the target DNA to the probe by ligation reaction;
removing the target DNA optionally under stringent conditions; and
detecting the signal of the labelled oligonucleotide.

Further, a method for multiple detection of different pathogens is provided, comprising the steps of providing a biochip; adding a target DNA from pathogens and a labelled detection oligonucleotide; hybridizing the target DNA to the probe by ligation reaction;
removing the target DNA optionally under stringent conditions; and
detecting the signal of the labelled oligonucleotide.

### Brief description of drawings

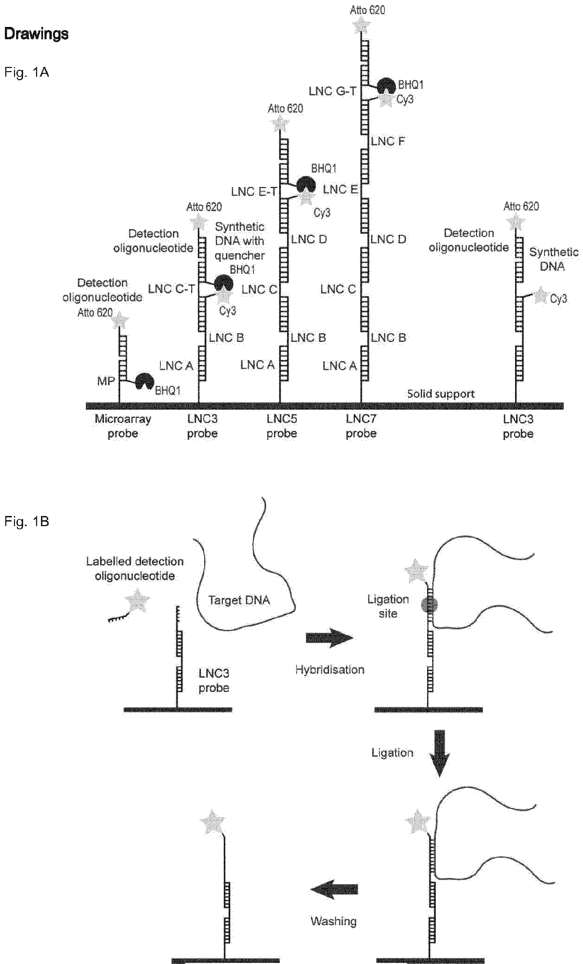

Fig. 1. Schematic illustration of the LNC probes and the reaction mechanism. (A) The LNC probes are formed by several individual oligonucleotides spotted simultaneously resulting in the terminal LNC X-T probe attached via hydrogen bridge bonds to the solid support. The terminal LNC probe binds to the DNA target. The synthetic DNA templates were modified with the quencher BHQ1 to monitor the DNA hybridisation behaviour. (B) A single enzymatic reaction comprising a ligation and a subsequent washing step is necessary for the DNA target detection. The DNA target is captured by the terminal LNC X-T probe and serves as template for the labelled detection oligonucleotide during the ligation reaction.

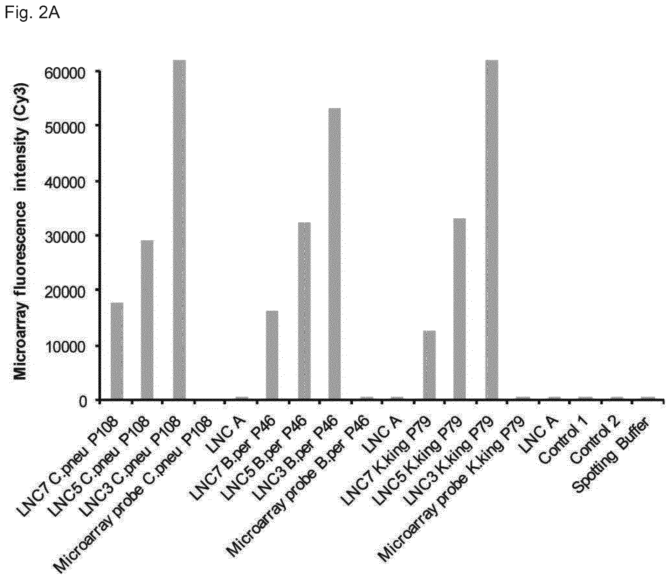

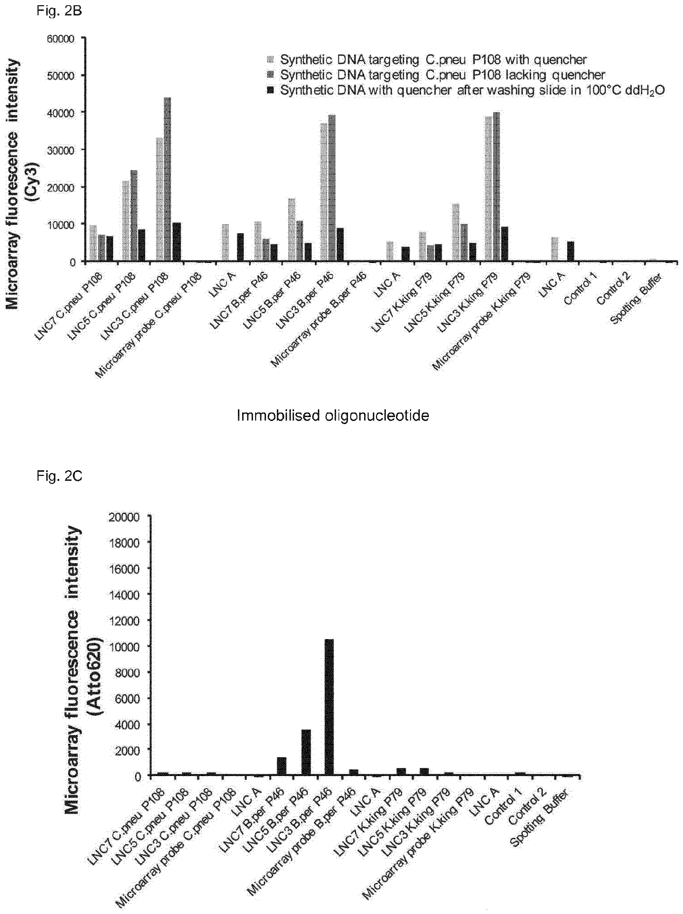

Fig. 2. Microarray results from experiments using different chain lengths of LNC probes. (A) The impact of the number of chain elements on the spotting efficiency was compared. Every complete LNC probe had a fluorescence conjugate, Cy3, attached to monitor the spotting efficiency. (B) The hybridisation behaviour of the target DNA to the LNC probes was compared in three different ligation experiments. First, a slide was analysed using a synthetic DNA as template comprising the quencher BHQ1 at its 3'-end. In a second experiment, synthetic DNA lacking BHQ1 was used. The LNC3 probe C.pneu P108 targeting the template C.pneu P108 showed lower signal intensities in the experiments using a quencher. However, the quenching effect was weaker than expected. Interestingly, the basal LNC A chain element produced a fluorescence signal in the Cy3-channel in combination with synthetic templates comprising BHQ1 although no Cy3 was attached. This phenomenon was investigated in a third experiment in which the Cy3 labelled LNC probes were removed from the slides prior the ligation reaction using synthetic templates comprising BHQ1. Despite the lack of the Cy3 fluorophore, all spots comprising the basal LNC A produced a fluorescence signal. (C) The impact of the number of chain elements on the sensitivity was compared. The LNC3 probes produced significantly higher signals in comparison to LNC5 and LNC7 probes.

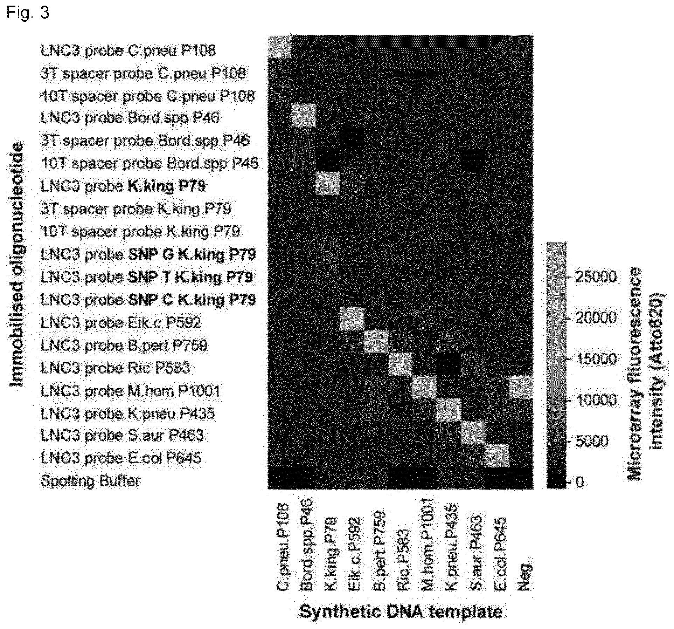

Fig. 3. Heatmap of the microarray signals from 11 experiments using synthetic DNAs as the templates in the ligation reaction. Two experiments with negative controls were included. On the vertical axes, the immobilised probes are named according to their corresponding target, with the number after the letter P in the probe name indicating the nucleotide position on the 16S rRNA gene. The probes in bold letters target a SNP in *K kingae.*

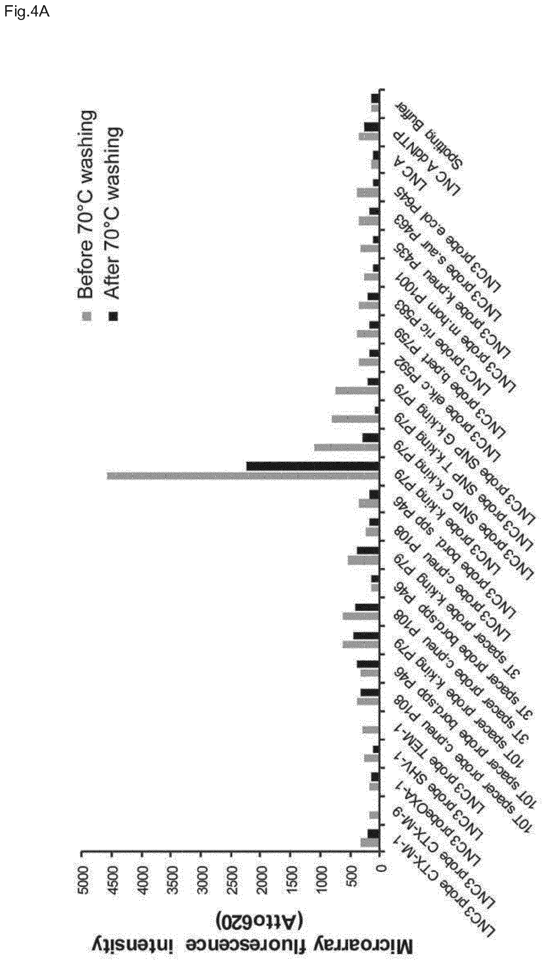

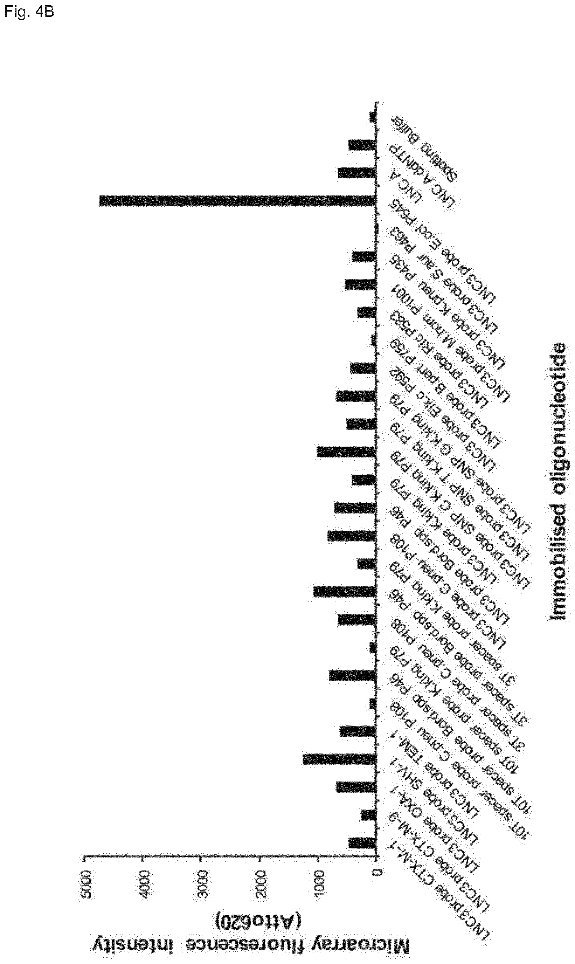

Fig. 4. Microarray results from 20-multiplex experiments. (A) In this experiment, the microarray slide was scanned directly after the ligation reaction and subsequently after a 70°C washing step in ddH₂O. (B) Results of a multiplex detection experiment using a genomic DNA extract from as little as 10⁷ cells of *E*. *coli* directly in the ligation reaction.

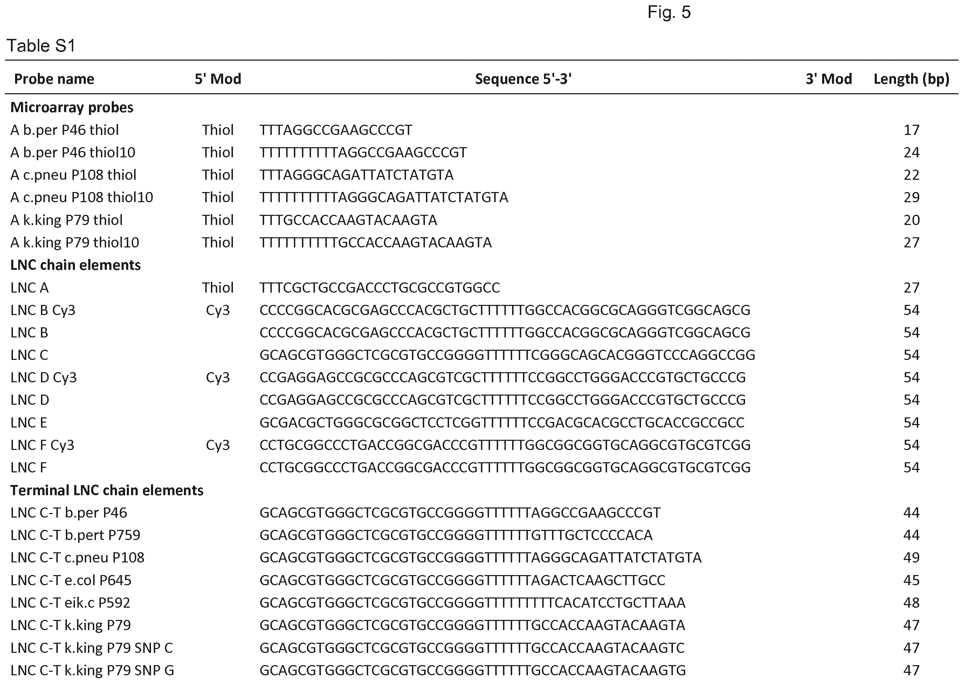

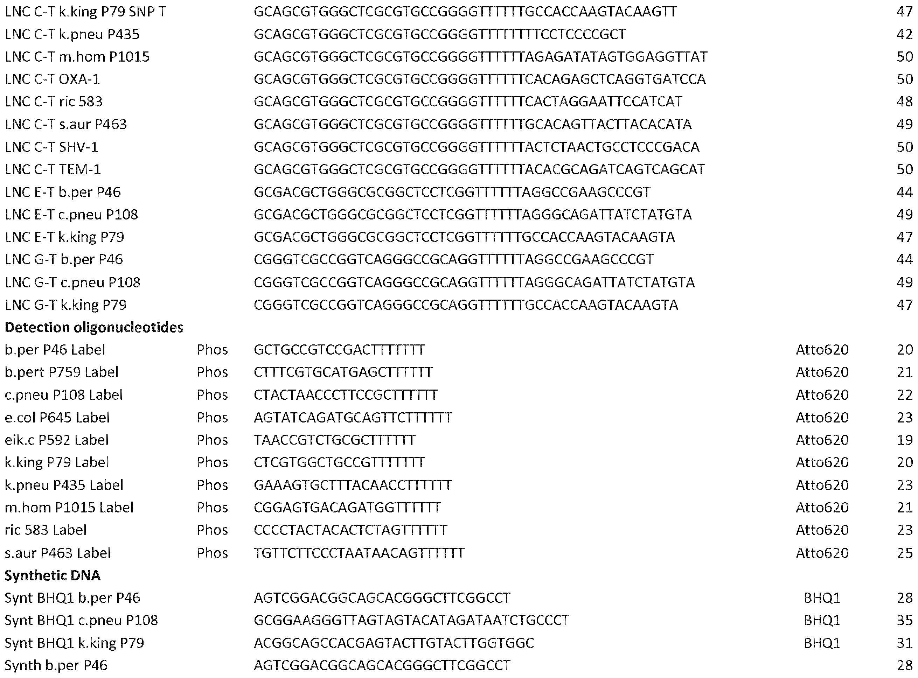

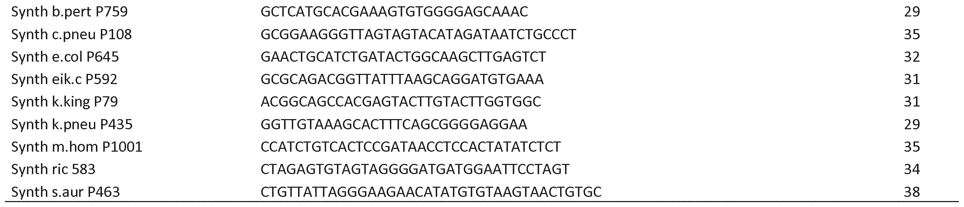

Fig. 5. Table S1 Oligonucleotides used for the manufacturing of the LNC probes and the evaluation experiments comparing the performance of conventional microarray probes with the LNC probes.

### Detailed description of the invention

Before describing in detail exemplary embodiments of the present invention, definitions important for understanding the present invention are given. As used in this specification and in the appended claims, the singular forms of "a" and "an" also include the respective plurals unless the context clearly dictates otherwise.

The term "solid substrate" according to the invention is a solid support, i.e. comprising a support material, which is mainly of non-liquid consistence and thereby allows for an accurate and traceable positioning of the LNCs on the support material. Preferably, said solid support is a planar support. An example of such a substrate is a solid material or a substrate comprising functional chemical groups, e.g. amine groups or amine-functionalized, aldehyde, thiol groups. A corresponding support material or substrate may be, for example, a non-porous substrate. Preferred among non-porous substrates is glass, silicone, fused silica, coated with a silane layer with functionalized groups, like, poly ethylene glycol, amine, epoxide, isothiocyanate, poly-L-lysine coated material, nitrocellulose, polystyrene, cyclic olefin copolymers, cyclic olefin polymers, polypropylene, polyethylene, polycarbonate etc. For example, glass or polystyrene may be used.

The term "substrate" as used herein refers to any suitable substrate known to the person skilled in the art. The substrate may have any suitable form or format, e.g. it may be flat, curved, e.g. convexly or concavely curved towards, it may be curled or comprise a wavelike format. It may also be organized in round shape structures, in the form of bead-like elements, or beads, or micro-carriers, which may, for example, be arranged in an array. The beads may overly a substrate ground or be fixed in the reaction area by connector elements like rods etc.

The term "microarray" as used herein refers to an ordered or random array on a solid substrate presented for interaction between antisense strand nucleotide sequences complementary to a target sequence in the array and potential interactors, i.e. DNA target nucleotides in the surrounding environment, e.g. a medium, a reaction solution, preferably a hybridization solution. A microarray may include any two- or three-dimensional arrangement of addressable regions, preferably a two-dimensional arrangement. A typical microarray may contain multiple spots, features, areas of individual immobilization or areas of individual molecular identity. For example, an array may contain more than 2, 5, 10, 50, 100, 500, 750, 1000, 1500, 3000, 5000, 10.000, 20.000, 40.000, 50.000, 70.000, 100.000, 200.000, 300.000, 400.000, 500.000, 750.000, 800.000, 1.000.000, 1.200.000, 1.500.000, 1.750.000, 2.000.000 or 2.100.000 spots, features, capture molecules or areas of individual immobilization or areas of individual molecular identity. These areas may be comprised in an area of less than about 20 cm², less than about 10 cm², less than about 5 cm², less than about 1 cm², less than about 1 mm², less than about 100 µm². In further embodiments the spot size within a microarray may be in a range of about 1 to 300 µm, e.g. have a size of 10, 50, 100, 150, 200, or 250 µm, or have any suitable value in between the mentioned values. In further embodiments, the probe densities may vary according to necessities. Examples of probe densities are densities between 10 to 100.000 probes per square µm, e.g. probe densities of 100, 250, 500, 1000, 1500, 2000, 10,000, 50,000 or 75,000 probes per square µm.

In specific embodiments such spots, features, or areas of individual immobilization may comprise any suitable coverage, e.g. coverage of 1, 1.5, 2, 3, 4, 5, 6, 7, 8, 9, 10 or more of one or more specific probes. Thus, a microarray may, for example, comprise 1, 2, 3, 4, 5, 6, 7, 8, 9, 10 or more copies of the same probes or different ones. Alternatively or additionally, it may comprise variations of a DNA molecule of interest, i.e. a target DNA molecule,, e.g. mismatch variants, variations with single or multiple nucleotide polymorphisms, different versions of single or multiple nucleotide polymorphisms.

The microarray typically comprises a solid substrate, on which one or more species of probes are immobilized.

The term "nucleic acid" as used herein refers to any nucleic acid known to the skilled person, preferably to DNA or RNA.

The term "complementary" as used in the present invention refers to the presence of matching base pairs in opposite nucleic acid strands. For example, to a nucleotide or base adenine (A) in a sense strand a complementary or antisense strand binds with a nucleotide or base thymine (T), or vice versa; likewise to a nucleotide or base guanine (G) in a sense strand the complementary or antisense strand binds with a nucleotide or base cytosine (C), or vice versa. This scheme of complete or perfect complementarity may, in certain embodiments of the invention, be modified by the possibility of the presence of single or multiple non-complementary bases or stretches of nucleotides within the sense and/or antisense strand(s), although 100% complementarity is preferred. However, to fall within the notion of a pair of sense and antisense strands, both strands may be completely complementary or may be only partially complementary, e.g. show a complementarity of about 80%, 85%, 90%, 91%, 92%, 93%, 94%, 95%, 96%, 97%, 98%, 99%, 99.5%, or 100% between all nucleotides of both strands. Non-complementary bases may comprise one of the nucleotides A, T, G, C, i.e. show a mismatch e.g. between A and G, or T and C.

Due to the complementary regions, the LNCs are connected via base pairing and thus the development of hydrogen bounds to form a double helix.

Specifically, the complementary sequences of the LNCs, specifically the complementary regions to other LNCs contain at least 50%, specifically at least 55%, specifically at least 60%, specifically at least 65%, specifically at least 70%, specifically at least 75%, more preferably at least 80% guanines and cytidines. Said GC rich regions are more stable than regions with low GC-content which provides increased thermo stability. Preferably have melting temperatures of about 80-90°C, specifically about 85°C. Specifically the GC-rich regions are on the 3'- and 5'-ends of the respective LNCs.

According to the invention, the complementary region comprises a length of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 nucleotides, specifically a length of between 10 to 50 nucleotides, specifically 15 to 40 nucleotides, specifically 20 to 30 nucleotides, more specifically about 25 nucleotides, more specifically 24 nucleotides. The optimal number of chain links can be determined by the skilled person using well known methods.

In other embodiments, the nucleic acid molecule may be present in a single stranded form. The term "single strand" as used herein means that the nucleic acid molecule shows no base pairing between two nucleic acid molecules. A single stranded form of a nucleic acid may be obtained by melting or denaturation of duplex nucleic acid molecules, e.g. by heating such molecules to temperatures of about 85°C to 100°C, preferably to 95°C. Temperature conditions may further be made dependent on the sequence of the nucleic acid, its length, or medium properties, such as the presence of salt, detergents etc. Alternatively, single stranded forms of a nucleic acid may be obtained by a *de novo* synthesis of nucleic acid molecules, e.g. according to suitable synthesis methods known to the person skilled in the art, or by suitable filtering or enrichment process, including the methods as envisaged by the present invention.

A "target sequence" or "sequence of interest" as used herein may be any suitable nucleic acid molecule, which allows a specific interaction between the target sequence and the respective complementary region of the LNC as described herein. A target sequence may typically be a genomic DNA, cDNA, mRNA, miRNA, siRNA or an amplified DNA/RNA product, or a synthetically produced nucleic acid, e.g. an oligonucleotide, or any grouping or combination thereof. Furthermore, the target sequences may be a specific allelic variant of these molecules, e.g. mutant or disease-associated forms, or an allelic variant, which is useful in diagnostic identifications. The target sequence to be used within the context of the present invention, in particular within the context of the probes as defined herein may be a single type of target sequence. For example a nucleic acid having a specific nucleotide sequence is used, or the target sequence may comprise a class of sequences, for example nucleic acids of varying lengths including or comprising a specific nucleotide sequence, or nucleic acids corresponding to various alleles of a particular gene, or nucleic acids comprising specific sequences and linker sequences or common primer binding site sequences etc.

Target sequences may be obtained or derived from samples. The present invention accordingly envisages any sample, which include target sequences as defined herein. Such samples may, for example, include samples derived from or comprising stool, whole blood, serum, plasma, tears, saliva, nasal fluid, sputum, ear fluid, genital fluid, breast fluid, milk, colostrum, placental fluid, amniotic fluid, perspirate, synovial fluid, ascites fluid, cerebrospinal fluid, bile, gastric fluid, aqueous humor, vitreous humor, gastrointestinal fluid, exudate, transudate, pleural fluid, pericardial fluid, semen, upper airway fluid, peritoneal fluid, fluid harvested from a site of an immune response, fluid harvested from a pooled collection site, bronchial lavage, urine, biopsy material, e.g. from all suitable organs, e.g. the lung, the muscle, brain, liver, skin, pancreas, stomach, etc., a nucleated cell sample, a fluid associated with a mucosal surface, hair, or skin. In addition, samples from environmental sources, e.g. water samples, meat or poultry samples, samples from sources of potential contamination etc. may be used.

"Target sequence" or "sequence of interest", " as used herein refer to either to a complete target sequence, for example the whole sequence of a pathogen or to parts of a target sequence in which not the entire target molecule, typically the entire single stranded molecule, is complementary to the respective sequence of the LNC, but wherein only a portion thereof is complementary and/or able to bind. This part or portion of said target molecule may have a minimal length of at least 5, 6, 7, 8, 9,10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 or 60 nucleotides. Furthermore, the respective complementary sequence of the LNC that is binding only to a part of a target sequence may have a minimal length of at least 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 or 60 nucleotides. In further additional or alternative embodiments, both interaction molecules, i.e. the respective complementary sequence of the LNC and a target sequence may, over their entire length, show a complementarity of about 30%, 33%, 35%, 40%, 45%, 50%, 55%, 60%, 70%, 75%, 80%, 85%, 90%, 91%, 92%, 93%, 94%, 95%, 96%, 97%, 98%, 99%, 99.5% or 100%. According to a preferred embodiment, the complementarity is at least 90%, specifically at least 95%, more specifically at least 99%, specifically 100%.

The term "immobilized" as used herein refers to the association of one or more LNCs to a supportive substrate via molecular interactions which position the LNC at a specific area of the substrate and concomitantly impede a detaching of the capture molecule, e.g. during washing, rinsing or interaction steps etc. Typically, such molecular interactions are based on covalent chemical bonds between structural elements or functional groups of the support material and the LNC, specifically the 5'ends of the LNC to be immobilized, e.g. corresponding functional groups of nucleic acids, as known to the person skilled in the art. The immobilization may, for example, be carried out by crosslinking the LNCs by heat or light, i.e. by forming molecular interactions or bonds that link both structural elements together under the influence or driven by the energy provided by an energy source like heat or light, or via a chemical immobilization.

A "chemical immobilization" may be an interaction between the support material and the LNC based on chemical reactions. Such a chemical reaction does typically not rely on the input of energy via heat or light, but can be enhanced by either applying heat, e.g. a certain optimal temperature for a chemical reaction, or light of certain wavelength. For example, a chemical immobilization may take place between functional groups on a support material and corresponding functional elements on the 5'-end of the LNCs coupled to the solid support. Such corresponding functional elements in the capture molecules may either be part of the chemical inventory of a molecule, or be additionally introduced. An example of such a functional group is a thiol group. Means and methods for such a chemical modification are known to the person skilled in the art. Functional groups, which impart a connecting reaction of the LNCs, are known to the person skilled in the art. An alternative reaction partner for LNCs to be immobilized may have to be chemically activated, e.g. by the activation of functional groups, available on the support material. The term "activated support material" relates to a material in which interacting or reactive chemical functional groups were established or enabled by chemical modification procedures as known to the person skilled in the art. For example, a substrate comprising carboxyl groups has to be activated before use. Furthermore, there are substrates available that contain functional groups that can react with specific moieties already present in the nucleic acids. Some of these reactions are enhanced by heat or UV. Examples are amine groups on the surface of the substrate, which can be bound to specific bases in the DNA.

Specifically, the LNCs are immobilized on the solid substrate covalently or via hydrogen bonds.

In particular disclosed herein is a solid substrate with a plurality of nucleotide probes immobilized thereto, wherein each probe contains at least three linear nucleotide sequence chains (LNCs) with complementary nucleotide regions which are able to build an interrupted double helix structure, wherein
a. a first LNC is immobilized to said solid substrate via the 5' end and has a nucleotide region complementary to a second LNC,
b. the second LNC is linked to one or more further LNC(s) via complementary nucleotide regions , and wherein
c. the terminal LNC comprises a nucleotide region complementary to a nucleotide sequence of interest.

Further described herein is a solid substrate with a plurality of nucleotide probes immobilized thereto, wherein each probe contains two linear nucleotide sequence chains (LNCs) building an interrupted double helix due to complementary nucleotide regions and wherein
a. a bottom LNC is directly immobilized to the solid substrate, has a nucleotide region complementary to second LNC and
b. a top LNC comprises a nucleotide region complementary to said bottom LNC and a nucleotide region complementary to a nucleotide sequence of interest.

Even more particularly, described is a solid substrate with a plurality of nucleotide probes immobilized thereto, wherein each probe contains more than two linear nucleotide sequence chains (LNCs) building an interrupted double helix due to complementary nucleotide regions and wherein
i. a bottom LNC is directly immobilized to the solid substrate comprising a nucleotide region complementary to a further LNC,
ii. one or more central LNCs, each containing two nucleotide regions complementary to LNCs, and
iii. a top LNC comprising a target specific antisense nucleotide sequence and a nucleotide region complementary to another LNC.

Reference to the terms "bottom", "central" and "top" shall illustrate the type of arrangement of the respective species of LNCs, thus the bottom LNC of the invention is characterized by a structure, specifically a 5'structure capable of being immobilized to the solid substrate and comprising a complementary sequence to a further LNC; the central LNC is characterized by comprising two complementary regions, wherein each of these regions are capable of building a helical structure with further LNCs having the respective antisense sequences; and a top LNC characterized by comprising a complementary sequence to a further LNC and a complementary sequence to a target sequence.

Specifically, each probe comprises 3, 4, 5, 6, 7, 8, 9, 10, 11, 12, 13, 14, 15 LNCs, specifically 3 to 10 LNCs, preferably 3 to 5 LNCs, preferred 3 LNCs. If 3 LNCs build a probe, the LNCs are of bottom, central and top species as described above. If more than three LNCs are present in the probe, the number of central LNCs is increased accordingly to increase the chain length of the probe. The sensitivity of the respective LNCs of the probe can be compared by the skilled person thus the optimized number of LNCs can be selected.

Said complementary sequences optionally may be separated by a spacer sequence. specifically said spacer sequences are of 1, 2, 3, 4, 5, 6, 7, 8, 9, 10, 11, 12, 13, 14 or 15 nucleotides length, specifically of about 3 to 10 nucleotides length, preferably 3 to 6 nucleotides, preferably said nucleotides are sequences of T.

The sensitivity of said LNC based detection system was at least 5-times, specifically at least 7.5-times, more preferred at least 10-times increased compared to microarray systems commercially provided and detecting the same target sequences.

The LNCs may comprise a nucleotide region complementary to a sequence of interest which further contains a fluorescence marker. Said marker may fluorescent labelling agent capable of being detected via fluorescence screening, specifically said marker may be selected from the group of DAPI, cyanine, AlexaFluor, ATTO, Dyomics, FAM, more specifically it may be Cy2, Cy3, Cy5, Cy7, specifically it is Cy3.

According to a further embodiment, the LNCs may also contain one or more quencher dyes for monitoring the DNA hybridization behavior. Such quencher molecules may be selected from the group of BHQ, Dabcyl, TAMRA, specifically it is a black hole quencher dye like BHQ1.

The solid substrate can be used for being part of a biochip containing the component of a microarray or equivalent thereof comprising the substrate with a plurality of probes according the invention.

According to an embodiment of the invention, a detection oligonucleotide is added and brought into contact with the probe.

The detection oligonucleotide specifically may be of a length of 1,2,3,4,5,6,7,8,9,10,11,12,13,14,15,16,17,18,19, 20 or more nucleotides, specifically of about 10 to 20 nucleotides, more specifically about 15 nucleotides.

Specifically, the detection oligonucleotide comprises a sequence complementary to the sequence of interest.

Said detection oligonucleotides may contain a phosphorylated 5'-end and/or a labelled 3'-end. Said labelling may be any label such as fluorescence molecules or biotin/streptavidin useful for detecting the binding of said oligonucleotide to the probe. Specifically a fluorescent dye is used, more specifically it is selected from Atto620, Atto532, Cy5, and Cy3.

For production of a biochip a solid substrate is provided and the probes are added and spotted onto the substrate.

The biochip may be part of a kit comprising the biochip and a detection oligonucleotide.

The biochip may be suitable for the detection and/or identification of a pathogen, specifically of bacterial origin, for genetic analysis of cells, specifically of eukaryotic cells, for analyzing antibiotics resistance genes, viruses and prokaryotic cells.

Herein disclosed is a method for detecting a nucleotide of interest comprising the steps of providing a system, for example a biochip, containing at least one probe, contacting said probes with a target sequence and a labelled detection oligonucleotide under conditions that allow hybridizing the target nucleotide to the probe by ligation reaction and the detection oligonucleotide; removing the target sequence under conditions sufficient to separate the target sequence; and detecting the signal of the labelled oligonucleotide.

Herein further disclosed is a method for multiple detection different sequences of interest like pathogens, comprising the steps of providing a system, for example a biochip, containing at least one probe, contacting said probes with a target sequence and a labelled detection oligonucleotide under conditions that allow hybridizing the target nucleotide to the probe by ligation reaction and the detection oligonucleotide; removing the target sequence under conditions sufficient to remove the target sequence; and detecting the signal of the labelled oligonucleotide.

### Examples

### Example 1 - Bacterial samples

The intracellular bacterial strains *Kingella kingae* (DSM 7536), *Bordetella pertussis* (DSM 5571), *Eikenella corrodens* (DSM 8340), *Bordetella bronchiseptica* (DSM 13414), *Rickettsia hoogstraalii*(DSM 22243), *Mycoplasma hominis* (DSM 19104) and *Chlamydophila pneumoniae* (ATCC VR2282) were ordered from the DSMZ and ATCC biobank, respectively. The lyophilised intracellular pathogens were resuspended in LB-medium prior DNA isolation. The pathogens *E. coli(EC10), K. pneumoniae* (85813) and *S. aureus* (SA8) were clinical isolates. These three isolates were grown in LB-medium at 37°C overnight prior DNA isolation. Then, 500 µl of the cell cultures were centrifuged at 8000 rpm for 10 minutes. The pellet was washed in 500 µl 1× PBS-buffer. This step was repeated once. Subsequently, the pellet was resuspended in 300 µl ddH₂O and disrupted using the Magna Lyser (Roche, Basel, Switzerland) at 6500 rpm for two times 30 seconds. The 30-seconds steps were interrupted by a 5 minutes pause. Then, the cells were thermally lysed at 95°C for 20 minutes. Finally, the suspension was centrifuged at 13000 rpm for 10 minutes. The supernatant was used directly in the subsequent experiments. The DNA extracts were quantified using an Epoch spectrometer (BioTek, Winooski, VT, USA) with final DNA concentrations that ranged from 2 ng/µl to 140 ng/µl.

Additionally, the PCR products of the 16S rRNA gene were used as DNA templates in the ligation evaluation experiments. The universal PCR primers 45F++ (GCY TAA YAC ATG CAA GTC GAR CG) and 783R (TGG ACT ACC AGG GTA TCT AAT CCT) were used to amplify the 16S rRNA gene. The Mastermix 16S Basic PCR Kit (Molzym, Bremen, Germany) was used according to the manufacturer's instructions.

### Example 2 - Oligonucleotides

The assay was designed to detect the ten above mentioned bacterial species using the 16S rRNA gene as phylogenetic marker. The discriminatory power of the new probe structures was evaluated characterising a SNP in *K. kingae.* Conventional microarray probes comprising 3T and 10T spacers were compared to the new probe structure, the linear chain probes (LNC probes). The oligonucleotides that were covalently immobilised had a thiol-modified 5'-end. The detection sequence of the microarray and LNC probes was identical. The LNC probes consisted of an alternating DNA double/single helix covalently attached at its 5'-end to a glass slide (Fig. 1A). Different chain sizes were designed to determine the optimal number of chain links. The chain elements comprise GC-rich regions at their ends with melting temperatures of ca. 85°C (Table S1). The mode of action of the LNC probes is illustrated in Fig. 1B. The detection oligonucleotide had a phosphorylated 5'-end and a fluorescently labelled 3'-end (Atto620). The DNA sequences of the detection oligonucleotides and synthetic DNA templates are listed in Table S1. First, synthetic DNA templates were used to evaluate the ligation process of each probe. A set of synthetic DNA templates had a modified nucleotide with a BHQ1 quencher at its 3'-end to test the hybridisation efficiency to the LNC probes (Fig. 1A). All nucleotide probes were ordered from Microsynth (Balgach, Switzerland).

### Example 3 -Surface modification and spotting

The slide manufacturing was based on a slightly modified protocol of Adessi *et al*. First, unmodified glass slides were cleaned by sonication in acetone for 10 minutes, immersed twice in ddH₂O and sonicated in 1 M NaOH for 10 minutes. Then, they were washed twice in ddH₂O and immersed in 1 M HCI overnight. Subsequently, the slides were washed twice in ddH₂O, rinsed with ethanol and air dried. The dried glass slides were silanized by immersion in a 0.5% solution of (3-aminopropyl)trimethoxysilane in dry acetone for 1 hour. Afterwards, they were washed three times in acetone for 5 minutes, immersed in ethanol and baked at 90°C for 50 minutes. To activate the surface with a cross-linker, the slides surface was incubated with a 2 mM solution of s-MBS (m-maleimidobenzyl-*N*-hydroxysulfo-succimide ester) in PBS (0.1 M NaH₂PO₄, 0.15 M NaCl, pH 7.2) for 5 hours at RT in a humid atmosphere. Then, the slides were immersed in PBS, ddH₂O and ethanol. The dry slide was ready for the spotting of the LNC probes.

The LNC and microarray probes were spotted using the Omnigrid contact arrayer (GeneMachines, San Carlos, CA, USA) and SMP 3 pins (TeleChem, Sunnyvale, CA, USA) at a final concentration of 5 µM per oligonucleotide in sterile filtrated 0.5x NaPi (0.05 M NaH₂PO₄, 0.075 M NaCl, pH 6.5). The different single oligonucleotides forming one LNC probe were pooled prior spotting. The adjusted air humidity was 60%. All subsequent washing steps were carried out in the dark. After spotting, the slides were incubated in a humid chamber at RT for 5 hours. Then, they were washed in 1× NaPi for 5 minutes and incubated with 10 mM mercaptoethanol in 1× NaPi for 1 hour. The slides were washed again in 1× NaPi for 5 minutes and immersed in a solution comprising 1.5 M NaCl and 10 mM NaH₂PO₄ (pH 7) for 10 minutes. Finally, the slides were washed in 5x SSC buffer (0.1%Tween) for 5 minutes, rinsed with 5x SSC, washed twice in ddH₂O for 2 minutes and centrifuged to dry. The dried slides were stored at -20°C.

### Example 4 - Ligation reaction and analysis

The ligation reaction was performed in a frame-seal incubation chamber with 25 µl capacity (Bio-Rad, Hercules, CA, USA). The reaction chamber was bond to the microarray slide, and the mixture was pipetted onto the slide. The reaction comprised a 300 nM mixture of each detection oligonucleotide in ampligase buffer (20 mmol/l Tris-HCI (pH 8.3), 25 mmol/l KCl, 10 mmol/L MgCl2, 0.5 mmol/l nicotinamide adenine dinucleotide (NAD), and 0.01% Triton® X-100), 7.5 units of ampligase (Epicentre, Madison, WI, USA), 6 µg of bovine serum albumin (BSA), and 1 µl of template DNA in a total volume of 30 µl. The ligation was incubated at 55°C for 1 hour. Subsequently, the slides were washed in 2× SSC comprising 0.1% sodium dodecyl sulfate (SDS) for 5 minutes, followed by washing in 0.2× SSC for 2 minutes and a final washing step in ddH2O for 1 minute. The slides were dried in a centrifuge and scanned using the Tecan Powerscanner (Männedorf, Switzerland). The data were analysed using GenePix Pro 6.0 and Microsoft Excel 2007.

### Example 5 - Spotting efficiency, stability of LNC probes and quencher phenomenon

Every LNC probe complex was fluorescently labelled using Cy3 to analyse the spotting efficiency and the correct formation of the immobilised probe structures (Fig. 1A). The formation efficiency of the LNC probe structures was dependent on the number of single-stranded DNA chain elements (Fig. 2A). The Cy3 signal intensities of the LNC7 probes had to be significantly lower in comparison to the LNC3 probes illustrating a lower density and more incomplete probe structures of the larger LNC probes. As a consequence, the sensitivity of the LNC7 probes was significantly lower than the one of LNC3 probes. Initially, the microarray slides were stored in 5x SSC buffer at 4°C based on the protocol of Adessi *et al.* However, a strong decrease in Cy3 signal intensity was observed after three months of storage (data not shown). Thus, new storage conditions were tested. The loss in signal intensity was minimal after three months if the dried slides were stored at -20°C.

Synthetic DNA templates comprising the quencher BHQ1 on their 3'-ends were used to test the hybridisation behaviour of the target DNA to the LNC probes (Fig. 1A). The quencher was meant to interact with the Cy3 fluorophore upon hybridisation of the synthetic DNA template to the LNC probe and quench the fluorescence signal. Interestingly, the tested synthetic templates comprising the BHQ1 quencher could inhibit the Cy3 signals of the LNC probes not significantly but instead produced unintended and unexplained fluorescence signals (532nm) at the microarray spots comprising only the probe LNC A which was lacking any fluorophores (Fig. 2B). The microarray spots comprising only the LNC A oligonucleotide were spotted as control to monitor the stability of the LNC complexes during the enzymatic reaction and to identify the potential migration of the LNC structures between different spots with the LNC A base. In identical experiments in which the synthetic DNA template had no quencher attached, no fluorescence signals were observed at the LNC A spots. To exclude any contribution of the Cy3 labelled LNC probes to this phenomenon, four slides were washed in boiling ddH₂O for ten minutes and subsequently scanned. As expected, no signals in the green channel were observed because the Cy3 labelled LNC probes were attached via hydrogen bonds to the surface, and thus, washed away upon this stringent treatment. However, after a subsequent ligation reaction using these slides and the synthetic DNA template with the BHQ1 quencher all spots comprising a LNC A oligonucleotide produced a fluorescence signal in the green channel (Fig. 2 B). In exclusion experiments leaving single components of the ligation mixture out, the synthetic template comprising BHQ1 was identified as source for the fluorescence signals in the green channel. A melting curve analysis in a real-time-PCR cycler was also performed to test the quenching capability of the dissolved LNC probe complex (data not shown). Interestingly, if the LNC probe was not immobilised to a solid support than significant quenching of the Cy3 signal could be observed.

### Example 6 - Optimal chain length

Three different sizes of LNC probes were tested ranging from three to seven oligonucleotides per structure. The internal Cy3 control of every LNC probe revealed that the spotting efficiency of the LNC3 probes was the highest. However, the impact of the LNC probe size on the specificity and sensitivity of the detection reaction had to be analysed. As expected due to the varying spotting efficiencies, the sensitivity of the reactions involving LNC3 probes was the highest (Fig. 2C). Additionally, the LNC3 probes showed slightly lower unspecific signals in detection reactions using PCR-products as DNA targets in comparison to LNC5 and LNC7 probes.

### Example 7 - Specificity of LNC probes

The specificity of the LNC probes was tested using synthetic DNA templates, PCR-products and genomic DNA from clinical isolates. The 16S rRNA gene served as phylogenetic marker to identify various pathogens. The capability of the LNC probes to differentiate SNPs was also tested. The evaluation experiments comprising synthetic DNA templates showed that the detection reaction using LNC3 probes is highly specific (Fig. 3). In comparison to the conventional microarray probes comprising the 3T and 10T spacer, the fluorescence signals at the LNC3 spots were higher by a factor of 10 or more. In the experiments using PCR products and genomic DNA, the conventional microarray probes did not show any significant fluorescence signals. Furthermore, it was possible to differentiate the SNPs without any stringent washing step. The possibility to conduct a stringent washing step (5 minutes in 70°C ddH₂O), and thus, to increase the specificity of the SNP detection was also demonstrated (Fig. 4A). During the washing step, all unligated detection oligonucleotides were removed from the immobilised probes resulting in a highly specific method. Finally, the specificity was tested using genomic DNA from clinical isolates of *E. coli.* The genomic DNA was directly added to the ligation reaction without any amplification step. The highly specific detection of as little as 10⁷ cells per reaction was possible within one hour reaction time (Fig. 4B).

### DISCUSSION

To our best knowledge, we report the first highly specific enzymatic multiplex DNA detection reaction on a planar solid support such as a microarray. In contrast to DNA polymerases, DNA ligases have a higher proof-reading capability. Furthermore, errors are not exponentially amplified such as in a PCR. Ligation-based multiplex reactions in liquid phase can detect up to 10,000 target genes. Thus, novel approaches aimed to translate these ligation reactions to solid supports and to increase the specificity of the on-chip methods. The simplest technique used a fluorescently labelled oligonucleotide and attached it covalently to a microarray probe. This same reaction mechanism was applied in the present work using the LNC probes. The main question prior primer design was why does the ligation reaction is highly specific if the components are dissolved in the buffer but becomes unspecific if oligonucleotides are immobilised. The experience from many unsuccessful approaches testing a wide range of different probe types, sequences, reaction mechanisms, etc. (data not shown) suggested that the immobilised detection probe has to have the same hybridisation behaviour as the dissolved oligonucleotides e.g. padlock probes. The covalent attachment of an oligonucleotide to a large planar surface might influence its Brownian motion, and thus, change the DNA-DNA hybridisation behaviour. As a consequence, we avoided to bind our detection probes covalently to the surface but aimed to emulate the natural hybridisation behaviour by the attachment via hydrogen bridge bonds to the slide. This approach proved to be successful because even the highly specific detection of SNP was possible. In contrast to conventional microarrays comparing the signal intensities of true and false positives, the LNC3 probes can highly specifically identify SNPs avoiding any false positive signals (Fig. 4A). This high specificity implicates that a much higher degree of multiplexing than presented in this work should be possible using the LNC3 probes.

Additionally, the major advantages of the novel method in comparison to others represent the simplicity and the speed. The commercially available multiplex detection methods need several hours to obtain results, which is especially critical in infectious disease diagnostics. The β-lactamase detection kits from Check-Points (Check-MDR CT101, CT102, CT103; Wageningen, Netherlands) need 6.5 hours after the DNA extraction and 5 steps with a total hands-on time of 2.5 hours. The multiplex detection system from Nanostring Technologies (Seattle, WA, USA) theoretically capable of single molecule detection comprises only 3 steps with a total hands-on time of 15 minutes but needs 2 days to obtain results. Faster, real-time PCR based systems are however limited to a few genetic targets. Additionally, several other high multiplexing techniques were presented. However, they are all inferior regarding speed and simplicity to the novel LNC3 probes based method. In summary, we report the first highly specific multiplex detection method on a planar solid support providing superior speed and simplicity.

## Claims

1. A solid substrate comprising a plurality of DNA probes, wherein each probe contains a number of linear nucleotide chains (LNCs) linked together by complementary nucleotide regions, wherein
a. a first LNC is immobilized to said substrate via the 5' end, and is linked to a second LNC;
b. the second LNC is linked to one or more further LNC(s) via complementary nucleotides regions, and wherein
c. the terminal LNC comprises a nucleotide region complementary to a nucleotide sequence of interest.

2. The solid substrate according to claim 1, wherein the first LNC is immobilized on the solid substrate covalently or via hydrogen bonds.

3. The solid substrate according to claim 2 wherein the immobilized LNC contains a thiol-modified 5'-end.

4. The solid substrate according to any one of claims 1 to 3, wherein the LNCs contain at least one spacer region between the complementary regions, preferably said spacer regions are of three to ten nucleotides length, preferably three to six nucleotides, preferably said nucleotides are thymines.

5. The solid substrate according to any one of claims 1 to 4, wherein the complementary region of the LNC probes contain at least 50%, preferably at least 60%, preferably at least 70%, more preferably at least 80% guanines and cytidines.

6. The solid substrate according to any one of claims 1 to 5, wherein the nucleotide sequence of interest is a pathogen sequence.

7. The solid substrate according to any one of claims 1 to 6, wherein the LNCs are connected via hydrogen bounds to form an interrupted double helix.

8. The solid substrate according to claims 1 to 7, wherein said probe comprises two to ten LNCs, preferably three to five LNCs, preferred three LNCs.

9. The solid substrate according to any one of claims 1 to 8, wherein the complementary region comprises 10 to 50 nucleotides, specifically 15 to 40 nucleotides, specifically 20 to 30 nucleotides, more specifically about 25 nucleotides.

10. A biochip or microarray comprising a solid substrate according to any one of claims 1 to 9 with a plurality of probes.

11. A kit comprising a biochip according to claim 10 and an optionally labelled detection oligonucleotide.

12. Use of a biochip according to claim 10 for the detection and/or identification of a pathogen, for genetic analysis of viruses, eukaryotic and prokaryotic cells.

13. A method for detecting a DNA of interest comprising the steps of providing a biochip according to claim 10;
adding a target DNA and a labelled detection oligonucleotide;
hybridizing the target DNA to the probe by ligation reaction;
removing the target DNA under optionally stringent washing conditions; and detecting the signal of the labelled oligonucleotide.

14. 'The method according to claim 13, wherein the DNA of interest is that of a pathogen and wherein different pathogens are detected simultaneously.

## Patentansprüche

1. Fester Träger, der eine Vielzahl von DNA-Sonden umfasst, wobei jede Sonde eine Anzahl von linearen Nukleotidketten (LNKs) enthält, die durch komplementäre Nukleotidregionen miteinander verbunden sind, wobei
a. eine erste LNK über das 5'-Ende auf dem Träger immobilisiert ist und mit einer zweiten LNK verbunden ist;
b. die zweite LNK über komplementäre Nukleotidregionen mit einer oder mehreren weiteren LNK(s) verbunden ist und wobei
c. die endständige LNK eine Nukleotidregion umfasst, die zu einer Nukleotidsequenz von Interesse komplementär ist.

2. Fester Träger nach Anspruch 1, wobei die erste LNK auf dem festen Träger kovalent immobilisiert ist oder über Wasserstoffbrückenbindungen.

3. Fester Träger nach Anspruch 2, wobei die immobilisierte LNK ein Thiol-modifiziertes 5'-Ende enthält.

4. Fester Träger nach einem der Ansprüche 1 bis 3, wobei die LNKs mindestens eine Abstandshalterregion zwischen den komplementären Regionen enthalten, vorzugsweise weisen die Abstandshalterregionen eine Länge von drei bis zehn Nukleotiden, vorzugsweise drei bis sechs Nukleotiden auf, wobei die Nukleotide vorzugsweise Thymine sind.

5. Fester Träger nach einem der Ansprüche 1 bis 4, wobei die komplementäre Region der LNK-Sonden mindestens 50%, vorzugsweise mindestens 60%, vorzugsweise mindestens 70%, bevorzugter mindestens 80% Guanine und Cytidine enthält.

6. Fester Träger nach einem der Ansprüche 1 bis 5, wobei die Nukleotidsequenz von Interesse eine Erregersequenz ist.

7. Fester Träger nach einem der Ansprüche 1 bis 6, wobei die LNKs über Wasserstoffbrückenbindungen zur Ausbildung einer unterbrochenen Doppelhelix verbunden sind.

8. Fester Träger nach den Ansprüchen 1 bis 7, wobei die Sonde zwei bis zehn LNKs umfasst, vorzugsweise drei bis fünf LNKs, bevorzugt drei LNKs.

9. Fester Träger nach einem der Ansprüche 1 bis 8, wobei die komplementäre Region 10 bis 50 Nukleotide umfasst, besonders 15 bis 40 Nukleotide, besonders 20 bis 30 Nukleotide, insbesondere etwa 25 Nukleotide.

10. Biochip oder Mikroarray, der einen festen Träger nach einem der Ansprüche 1 bis 9 mit einer Vielzahl von Sonden umfasst.

11. Kit, der einen Biochip nach Anspruch 10 und ein wahlweise markiertes Nachweisoligonukleotid umfasst.

12. Verwendung eines Biochips nach Anspruch 10 zum Nachweis und/oder zur Identifizierung eines Erregers, zur genetischen Analyse von Viren, eukaryotischen und prokaryotischen Zellen.

13. Verfahren zum Nachweis einer DNA von Interesse, das die folgenden Schritte umfasst:
Bereitstellen eines Biochips nach Anspruch 10;
Hinzufügen einer Ziel-DNA und eines markierten Nachweisoligonukleotids;
Hybridisieren der Ziel-DNA an die Sonde durch Ligationsreaktion;
Entfernen der Ziel-DNA unter wahlweise stringenten Waschbedingungen; und
Nachweisen des Signals des markierten Oligonukleotids.

14. Verfahren nach Anspruch 13, wobei die DNA von Interesse die eines Erregers ist und wobei verschiedene Erreger gleichzeitig nachgewiesen werden.

## Revendications

1. Substrat solide comprenant une pluralité de sondes d'ADN, dans lequel chaque sonde contient un certain nombre de chaînes nucléotidiques linéaires (CNL) liées ensemble par des régions nucléotidiques complémentaires, dans lequel
a. une première CNL est immobilisée sur ledit substrat par l'intermédiaire de l'extrémité 5', et est liée à une deuxième CNL ;
b. la deuxième CNL est liée à une ou plusieurs autres CNL par l'intermédiaire de régions nucléotidiques complémentaires, et dans lequel
c. la CNL terminale comprend une région nucléotidique complémentaire d'une séquence nucléotidique d'intérêt.

2. Substrat solide selon la revendication 1, dans lequel la première CNL est immobilisée sur le substrat solide de manière covalente ou par l'intermédiaire de liaisons hydrogène.

3. Substrat solide selon la revendication 2, dans lequel la CNL immobilisée contient une extrémité 5' modifiée par un thiol.

4. Substrat solide selon l'une quelconque des revendications 1 à 3, dans lequel les CNL contiennent au moins une région d'espacement entre les régions complémentaires, de préférence lesdites régions d'espacement ont une longueur de trois à dix nucléotides, de préférence de trois à six nucléotides, de préférence lesdits nucléotides sont des thymines.

5. Substrat solide selon l'une quelconque des revendications 1 à 4, dans lequel la région complémentaire des sondes de CNL contiennent au moins 50 %, de préférence au moins 60 %, de préférence au moins 70 %, plus préférablement au moins 80 % de guanines et de cytidines.

6. Substrat solide selon l'une quelconque des revendications 1 à 5, dans lequel la séquence nucléotidique d'intérêt est une séquence de pathogènes.

7. Substrat solide selon l'une quelconque des revendications 1 à 6, dans lequel les CNL sont reliées par l'intermédiaire de liaisons hydrogène pour former une double hélice interrompue.

8. Substrat solide selon l'une quelconque des revendications 1 à 7, dans lequel ladite sonde comprend de deux à dix CNL, de préférence de trois à cinq CNL, préférablement trois CNL.

9. Substrat solide selon l'une quelconque des revendications 1 à 8, dans lequel la région complémentaire comprend de 10 à 50 nucléotides, spécifiquement de 15 à 40 nucléotides, spécifiquement de 20 à 30 nucléotides, plus spécifiquement environ 25 nucléotides.

10. Puce à ADN ou microréseau comprenant un substrat solide selon l'une quelconque des revendications 1 à 9 avec une pluralité de sondes.

11. Kit comprenant une puce à ADN selon la revendication 10 et éventuellement un oligonucléotide de détection marqué.

12. Utilisation d'une puce à ADN selon la revendication 10 pour la détection et/ou l'identification d'un pathogène, pour une analyse génétique de virus, de cellules eucaryotes et procaryotes.

13. Méthode de détection d'un ADN d'intérêt comprenant les étapes de fourniture d'une puce à ADN selon la revendication 10 ;
ajout d'un ADN cible et d'un oligonucléotide de détection marqué ;
hybridation de l'ADN cible à la sonde par une réaction de ligation ;
retrait de l'ADN cible dans des conditions de lavages éventuellement rigoureuses ; et détection du signal de l'oligonucléotide marqué.

14. Méthode selon la revendication 13, dans laquelle l'ADN d'intérêt est celui d'un pathogène et dans laquelle différents pathogènes sont détectés simultanément.
